(19) Europäisches Patentamt
European Patent Office
Office européen des brevets

(11) Veröffentlichungsnummer: **0 190 442**
**A1**

(12) # EUROPÄISCHE PATENTANMELDUNG

(21) Anmeldenummer: **85115828.7**

(51) Int. Cl.[4]: **C 07 J 9/00**

(22) Anmeldetag: **12.12.85**

(30) Priorität: **14.12.84 DE 3445650**

(43) Veröffentlichungstag der Anmeldung:
**13.08.86 Patentblatt 86/33**

(84) Benannte Vertragsstaaten:
**AT BE CH DE FR GB IT LI LU NL SE**

(71) Anmelder: **Klemke, Rudolf Erich, Dipl.-Chem.**
**Grosswiesenstrasse 18**
**D-7763 Öhningen(DE)**

(72) Erfinder: **Klemke, Rudolf Erich, Dipl.-Chem.**
**Grosswiesenstrasse 18**
**D-7763 Öhningen(DE)**

(74) Vertreter: **Ratzel, Gerhard, Dr.**
**Seckenheimer Strasse 36a**
**D-6800 Mannheim 1(DE)**

(54) **Verfahren zur Darstellung C7-hydroxylierter Cholesterole und Desmosterole.**

(57) Die vorliegende Erfindung betrifft ein Verfahren zur Darstellung von $\Delta^5$-Cholesten-3ß, 7α, ß-diolen (7α, ß-Hydroxycholesterolen) und/oder $\Delta^{5,25}$-Cholestadien-3β, 7α, β-diolen(7α, β-Hydroxy-desmosterolen), das dadurch gekennzeichnet ist, daβ die als Zwischenstufen verwendeten 3β-Acetoxy-7-keto-$\Delta^5$-cholestene bzw. -$\Delta^{5,25}$-cholestene unter Verwendung von tert.-Butylchromat aus Cholesterol bzw. Desmosterol synthetisiert werden.

EP 0 190 442 A1

Croydon Printing Company Ltd.

# Verfahren zur Darstellung $C_7$-hydroxylierter Cholesterole und Desmosterole

Die vorliegende Erfindung betrifft ein Verfahren zur Darstellung von $C_7$-hydroxylierter Cholesterole und Desmosterole.

Die Darstellung solcher Diole ist in der Literatur beschrieben: Windaus, Lettre und Schenk, Liebigs Annalen 520, 98 (1935) und O. Wintersteiner, W. L. Ruigh, J. Amer. Chem. Soc. 64 , 2435 (1942), wobei 7-Keto-cholesteryl-acetat nach Meerwein-Ponndorf mit Aluminium-iso-propylat in 80 %-iger Ausbeute zum 7α -Hydroxy-Epimeren und in 20 %-iger Ausbeute zum 7ß-Hydroxy-Epimeren umgesetzt wird.

Die Darstellung des als Ausgangsmaterial verwendeten 7-Keto-cholesteryl-acetats ist dabei bezüglich der Ausbeuten bis heute unbefriedigend. Die bekannten Verfahren von 1) A. Windaus, H. Lettre, Fr. Schenk, Liebigs Ann. 520, 98 (1935), 2) L.F. Fieser, M. Fieser, R.N. Chakravarti, J. Amer.Chem. Soc. 71, 2226 (1949) und 3) Louis Fieser, J. Amer. Chem. Soc. 75, 4394 (1953) liefern der Reihe nach lediglich 28 %, 33 % bzw. 37 % der Theorie an 7-Keto-cholesteryl-acetat.

Zwecks Erzielung einer höheren Ausbeute an diesem wichtigen Zwischenprodukt wurde die $C_7$-Oxidation des Cholesteryl- bzw. Desmosteryl-acetats erfindungsgemäß nicht mit Chrom-VI-oxid ($CrO_3$), sondern mit tert. Butylchromat vorgenommen, wie dieses von K. Heusser und Wettstein, Helv. chim. Acta 35, 284-294 (1952) beschrieben und von C. W. Marshall, Richard E. Ray, Ivar Laos und Byron Riedl, J. Amer. Chem. Soc. 79, 6308-6313 (1957) zur Darstellung von 3ß-Acetoxy-pregnan-5-en-7,20-dion aus 3ß-Acetoxy-pregnan-5-en

(Pregnenolon-acetat) verwendet wurde. Auf diese Weise ließ sich die Ausbeute an 7-Keto-cholesteryl-acetat auf über 62,3 % der Theorie steigern.

Eine weitere wichtige erfindungsgemäße Verbesserung besteht in der Verschiebung des Verhältnisses von der 7α - zur 7ß-Verbindung von 80% : 20 % auf 25 % : 75 % zu Gunsten des 7ß-Epimeren, wodurch dessen Zugänglichkeit eine wesentliche Ausbeutesteigerung erfährt. Dieses Ergebnis wurde durch Reduktion der 7-Keto-Gruppe statt mit Aluminium-isopropylat durch die Verwendung von Lithium-Aluminium-Hydrid bzw. entsprechender Borhydride erzielt, die wesentlich stereospezifischer wirken als Aluminium-isopropylat.

Alle dieser Verfahrensverbesserungen dienen dem Zweck, das 7ß-Hydroxy-cholesterol bzw. das 7ß-Hydroxy-desmosterol als untoxische, da körpereigene, Immun-Modulatoren und damit als untoxische Arzneimittel zur Behandlung von Krebserkrankungen besser zugänglich zu machen. Während das 7α -Hydroxy-cholesterol in der Leber gebildet wird und keinerlei physiologische Wirkung besitzt, wird das 7ß-Hydroxy-cholesterol in der Thymus-Drüse aller Säugetiere als universelle Signalsubstanz der körpereigenen Immunabwehr gebildet. Seine Wirkung, die ausschließlich auf maligne entartete Zelloberflächen gerichtet ist, verdankt es dem Umstand, daß es von der LDL (low density lipoproteins), die für den lebenswichtigen Transport von Cholesterol ins Zellinnere und zum Aufbau der Zellmembranen verantwortlich sind, unspezifisch gebunden, und von diesen, vermutlich via die NK-Zellen (natural killer cells), auf die Zellmembranen von entartetem Gewebe, insbesondere Krebszellen, transferiert wird. Da die Rezeptoren der LDL auf Krebszelloberflächen, im Gegensatz zu normalen Somazellen, nach außen gestülpt sind, wodurch diese eine räumliche Strukturveränderung erfahren haben,

bewirkt das 7ß-Hydroxy-cholesterol bzw.-desmosterol eine Blockierung solchermaßen veränderter Porenöffnungen, vergleichbar mit dem Stopfen einer Flasche, wodurch die Krebszelle von der Zufuhr des lebenswichtigen Cholesterols abgeschnitten wird. Als Folge davon baut sich im Inneren der Krebszelle ein osmotischer Überdruck auf, der schließlich zur kolloid-osmotisch induzierten Ruptur der Krebszelle führt, in deren Verlauf das Zytoplasma der Krebszellen herausgepreßt wird. Die Krebszelle hat aufgehört zu existieren.

Diesen Vorgang, der nur ca. 8 bis 10 Minuten dauert, hat Alex Matter (Microcinomatographic and electron microscopic analysis of target cell lysis induced by cytotoxic T lymphocytes, Immunology 36, 179 - 190 (1979) mikoskopisch dokumentiert, ohne indessen über die chemische Natur des körpereigenen Wirkstoffes eine Aussage zu machen.

Das 7ß-Hydroxy-cholesterol wurde 1976 erstmals von Klemke (unveröffentlichte Arbeiten) neben Progesteron, 11ß-Hydroxy-progesteron, Cortexon und 7-Keto-cholesterol mittels der Antimon-trichloid-Reaktion auf Steroide im Thymus nachgewiesen. Später haben Reisch und El Shakary, Scientia Pharmaceutica 50 , 75-78 (1982) diesen Befund bestätigt, nachdem zuvor die Arbeitsgruppe um J. P. Beck in Straßburg, J. Chem. Res. (S) 1977, 217 - 219, gefunden hatte, daß das 7ß-Hydroxy-cholesterol die antiproliferierend wirksame Aktivsubstanz einer uralten chinesischen Droge, der Bombyx cum Botryte, einer durch einen mikroskopischen Pilz (Botrytis bassiana Balls) abgetötete Seidenraupe (Bombyx mori), dargestellt. Nähere Einzelheiten dazu siehe die Buch-Neuerscheinung: TUMOSTERON ® im Verlag für Medizin Heidelberg, voraussichtlich Mitte 1985.

Mit dem $\Delta^5$-Cholesten-3ß, 7ß-diol ist eine biochemische Signalsubstanz des körpereigenen Immunabwehr-Systems entdeckt worden, die im Gegensatz zur herkömmlichen zytotoxischen Behandlung von Krebserkrankungen, völlig untoxisch und spezifisch in der Lage ist, Krebszellen aller Phänotypen selektiv zu eliminieren, und zwar ohne dabei gesunde Zellen in Mitleidenschaft zu ziehen.

Die einzelnen Verfahrensschritte gliedern sich wie folgt:

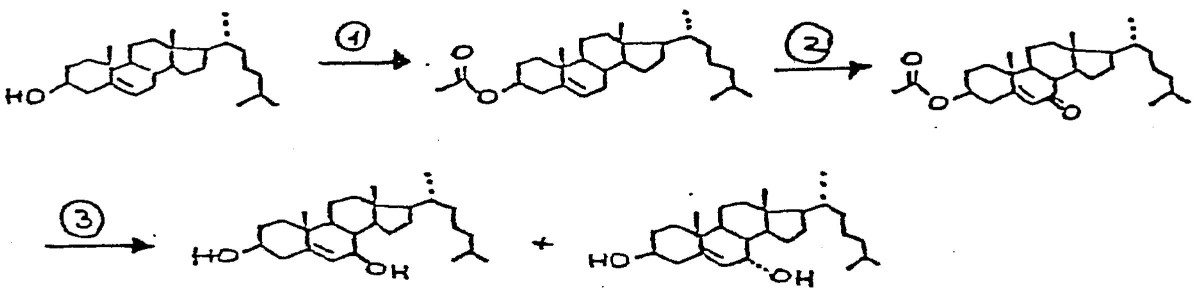


Die Erfindung wird im folgenden anhand von Ausführungsbeispielen weiterhin erläutert:

<u>Beispiel I:</u> <u>Darstellung von Cholesteryl-acetat</u>
(3ß-Acetoxy- $\Delta^5$-cholesten)

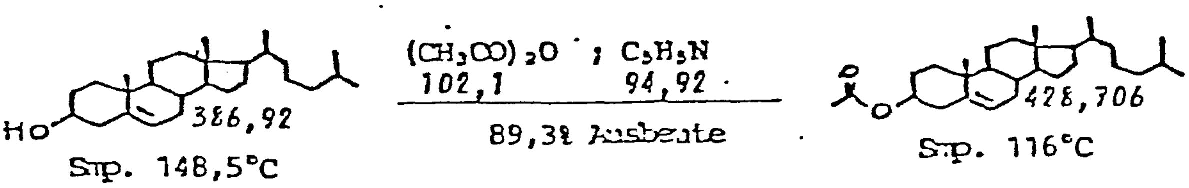

48,5 g (0,125 Mol) Cholesterol wurden mit 50 ml (0,5 Mol) Acetanhydrid und 50 ml Pyridin (0,6 Mol) versetzt und drei Stunden lang unter Rückfluß erhitzt ($CaCl_2$-Rohr), wobei sich die Reaktionsmischung allmählich dunkel färbt. Nach dem Abkühlen war der Ansatz im Reaktionskolben erstarrt. Durch gelindes Erwärmen wurde er wieder verflüssigt und in dünnem Strahl auf Eiswasser gegossen. Während des Rührens schmolz das Eis (ca. 500g) innerhalb von ca. 10 Minuten. Das ausgefallene Material wurde abgesaugt, mit $H_2O$ gewaschen und in einem (1) Liter $H_2O$ suspendiert. Die Suspension wurde mit 10%iger HCl angesäuert, abgesaugt und bis zur neutralen Reaktion des Waschwassers auf der Nutsche gründlich mit $H_2O$ gewaschen. Die Rohausbeute an feuchtem Umsetzungsprodukt betrug 95,6 g.

Anmerkung: Selbst nach 6 stündigem Trocknen bei 80°C im Trockenschrank betrug die Restfeuchtigkeit immer noch ca. 10 g $H_2O$; es wurden 62,8 g rohes Cholesteryl-acetat erhalten (theoretisch 53,6 g).

Das feuchte Rohprodukt wurde aus 700 ml Methanol/Äther (2 : 1) umkristallisiert. Ausbeute: 41 g (= 76,5 % d. Th.; Smp. 115,5°C).

Die Mutterlauge wurde auf 500 ml eingeengt, wobei eine Nachfällung eintritt. Nach drei (3) stündigem Stehen bei -1°C im Kühlschrank wurde abgesaugt.
Ausbeute: 5,6 g(= 10% d.Th.); Smp. 112-114°C. Damit erhöht sich die Ausbeute auf 86,9 % d.Th. .
Erneutes Einengen der Mutterlauge ergabe 1,5 g bräunlich-weißer Kristalle vom Smp. 106-110°C. Diese wurden aus 200ml $Ch_3OH$ umkristallisiert und ergaben 1,3 g (= 2,4 % d.Th.); Smp. 114°C. Damit erhöht sich die Gesamtausbeute auf 89,3 % der Theorie.

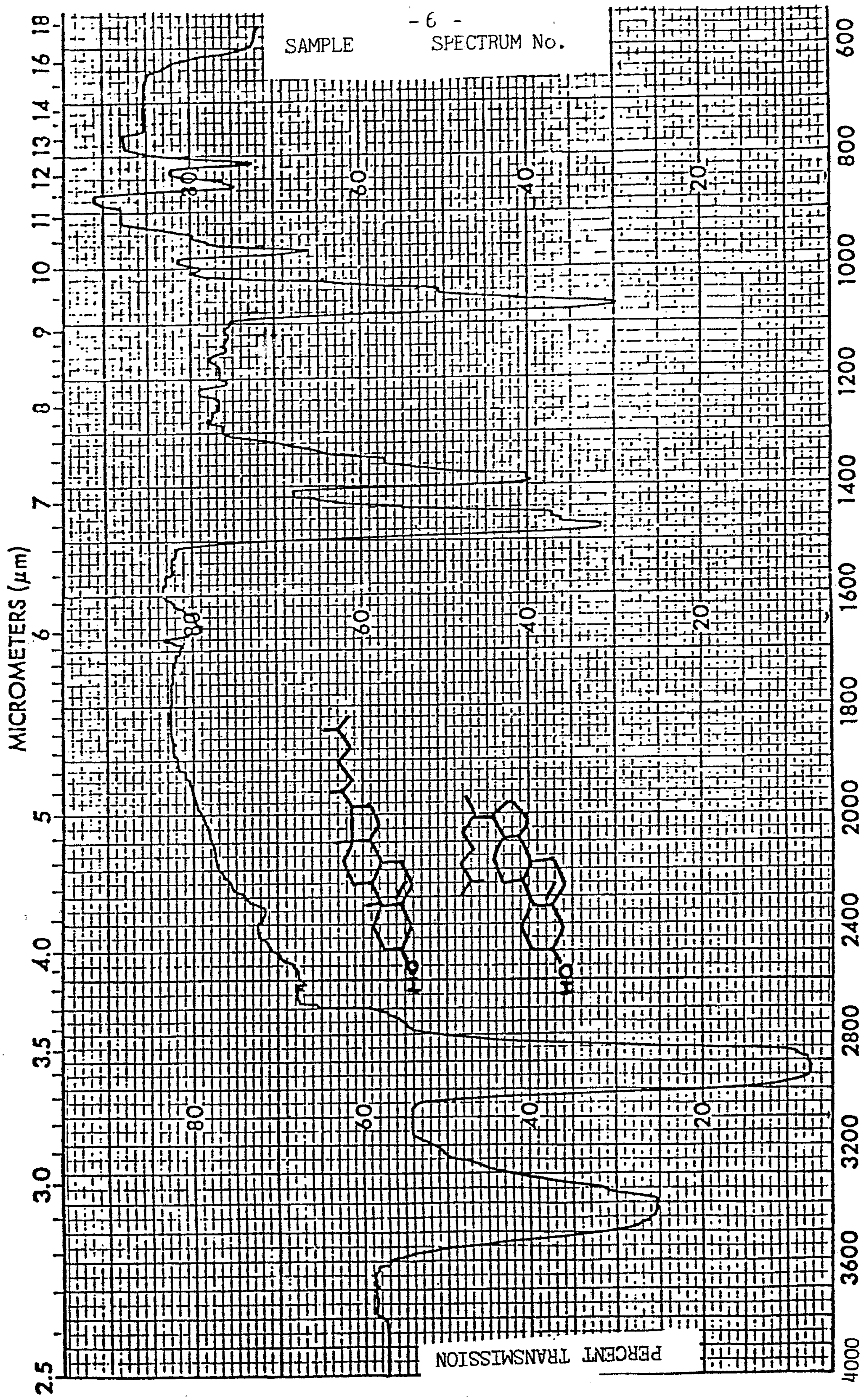
SAMPLE
SPECTRUM No.
MICROMETERS (μm)
FREQUENCY (CM⁻¹)
PERCENT TRANSMISSION

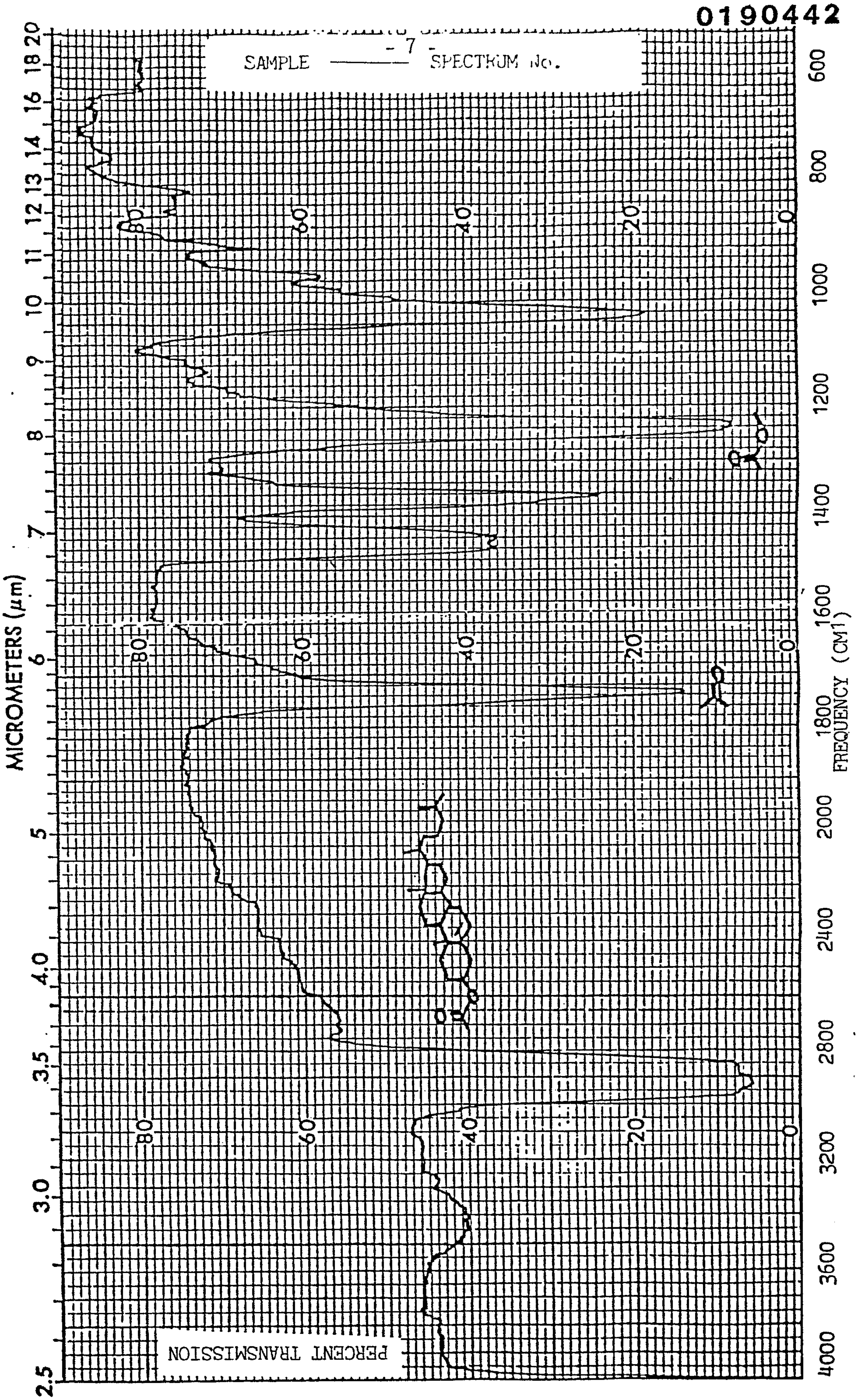
SAMPLE
SPECTRUM No.
MICROMETERS (μm)
FREQUENCY (CM⁻¹)
PERCENT TRANSMISSION

Beispiel II:

Darstellung von 7-Keto-cholesteryl-acetat
(7-Oxo-$\Delta^5$-cholesten-3ß-ol-acetat;
3ß-Acetoxy-$\Delta^5$-cholesten-7-on)

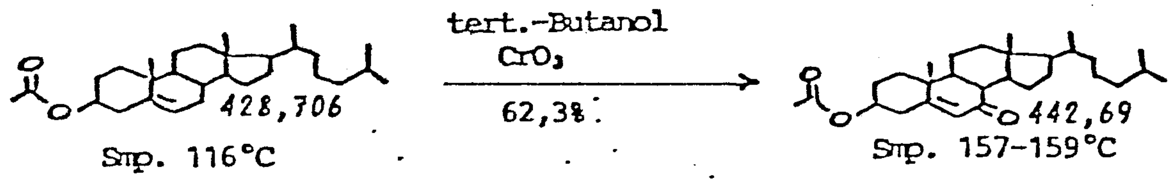


Smp. 116°C

Smp. 157-159°C

Das verwendete ter.-Butylchromat wurde unter Abänderung der Vorschrift von K.Heusser und Wettstein: Helv. chim. Acta 35,284-294 (1952) folgendermaßen hergestellt:

Darstellung von tert.-Butylchromat-Lösung:

In einem 500 ml Becherglas wurden 187,2 g (2,5 Mol) tert.-Butanol vom Smp. 24,5 °C auf 28°C erwärmt und geschmolzen. Zu dieser Schmelze wurden unter Verwendung eines Thermometers als Rührstab 74 g (= 0,74 Mol) $CrO_3$ portionsweise eingetragen. Um die Reaktionstemperatur unter 30°C zu halten, ist gelegentliches Kühlen mit Eiswasser erforderlich. Das flüssige Reaktionsgemisch wurde im Scheidetrichter mit 520 ml $OCl_4$ verdünnt und über Nacht sich selbst überlassen. Dies ist wichtig, denn die Lösung muß klar werden. Am nächsten Morgen wurde die obere dunkle wässrige Phase abgetrennt. Die klare $OCl_4$-Lösung wurde mit 50 g wasserfreiem $Na_2SO_4$ getrocknet, filtriert und das $Na_2SO_4$ mit 320 ml $OCl_4$ nachgewaschen. Die vereinigten $OCl_4$-Lösungen wurden anschließend im Vakuum bei einer Wasserbad-Temperatur von 40 - 45°C auf 400 ml eingeengt, wobei überschüssiges tert.-Butanol zusammen mit $OCl_4$ azeotrop überdestilliert. Diese Lösung ist im Kühlschrank bei -1°C mindestens einen Monat lang unverändert haltbar.

Darstellung von 3ß-Acetoxy- $\Delta^5$-cholesten-7-on:

Anmerkung: Zwecks Erzielung einer möglichst hohen Ausbeute wurde das Verfahren von C.W. Marshall, Richard E.Ray, Ivar Laos und Byron Riedl: J.Amer. chem. Soc. 79, 6308-6313 (1957) zur Darstellung von 3ß-Acetoxy-pregnan-5-en-7,2o-dion aus 3ß-Acetoxy-pregnan-5-en (Pregnenolon-acetat) in zweckmäßiger Weise abgewandelt. Die Verfahren von L.F. Fieser, M.Fieser, R.N. Chakravarti, J.Amer. chem.Soc. 71, 2226 (1949); A.Windaus, H.Lettre, Fr.Schenk, Liebigs Ann. 520,98 (1935); Louis Fieser J.Amer. chem. Soc. 75, 4394 (1953), ergeben lediglich 33%, 28% bzw. 37,6% Ausbeute. Mit folgendem Verfahren wurden jedoch 62,3 %. Ausbeute erzielt:

42,8 g (0,1 Mol) Cholesterylacetat wurden in 150 ml $OCl_4$ gelöst und in einem 2000 ml-Dreihalskolben mit Rückflußkühler, Thermometer und Tropftrichter auf 80°C erwärmt. Die Lösung beginnt zu sieden. Dann wurde unter kräftigem Rühren innerhalb von 30 Minuten eine Mischung aus 400 ml obiger tert.-Butylchromat-Lösung, 90 ml $CH_3OOOH$ und 36 ml $Ac_2O$ zugetropft. Anschließend wurde zehn (10) Stunden lang bei 80°C weitergerührt und zuletzt auf 20°C abgekühlt.

Unter Eiskühlung wurden sodann 60 g Oxalsäure gelöst in 600 ml $H_2O$, während 45 Minuten und kräftigem Rühren eingetropft. Wenn bei der Herstellung der tert.-Butylchromat-Lösung sorgfältig verfahren wurde, wird die Reaktionsmischung nicht dick. Die Reaktionstemperatur stieg dabei nie über 15°C. Genau 15 Minuten nach der Zugabe dieser wässrigen Oxalsäure-Lösung wurden 42 g feste Oxalsäure unter kräftigem Rühren portionsweise eingetragen (Vorsicht, kann etwas schäumen). Es wurde noch zwei (2) Stunden nachgerührt. Die Lösung ist tief dunkelbraun; die Temperatur der Reaktionslösung beträgt zum Schluß 20°C.

Nach Separation im Scheidetrichter wurde die gelb-grüne $OCl_4$-Lösung durch ein Faltenfilter in einen Erlenmeyer-Kolben filtriert und die wässrige Phase im Scheidetrichter nochmals mit 200 ml $OCl_4$ ausgeschüttelt. Die vereinigten $OCl_4$-Lösungen wurden der Reihe nach mit 500 ml $H_2O$, dann mit 500 ml einer gesättigten $NaHCO_3$-Lösung und zuletzt nochmals mit 500 ml $H_2O$ nachgewaschen, über $Na_2SO_4$ filtriert, das $Na_2SO_4$ mit $OCl_4$ nachgewaschen und die klare grüne Lösung auf dem Wasserbade zur Trockne gedampft.

Der Rückstand wurde am Rückflußkühler in 500 ml siedendem Äther gelöst. Die Lösung ist tiefbraun, fast schwarz. Nach Stehenlassen im Kühlschrank bei -1°C über Nacht, wurden 15,4 g Rohprodukt erhalten. Acht (8) Stunden später wurden erneut 7,5 g weißer Kristalle gewonnen. Durch Einengen der Mutterlauge auf ca. 150 ml und mehrstündigem Stehen im Kühlschrank wurden schließlich nochmals 5,8 g Rohprodukt erhalten.

Die vereinigten Kristallfraktionen wurden auf der Nutsche mit wenig eiskaltem Äther gewaschen (oder aus ca. 250 ml Äther umkristallisiert) und ergaben insgesamt 26,7 g reines 7-Keto-cholesteryl-acetat vom Smp. 158,5 °C. Damit errechnet sich die Gesamtausbeute auf 62,3 % der Theorie.

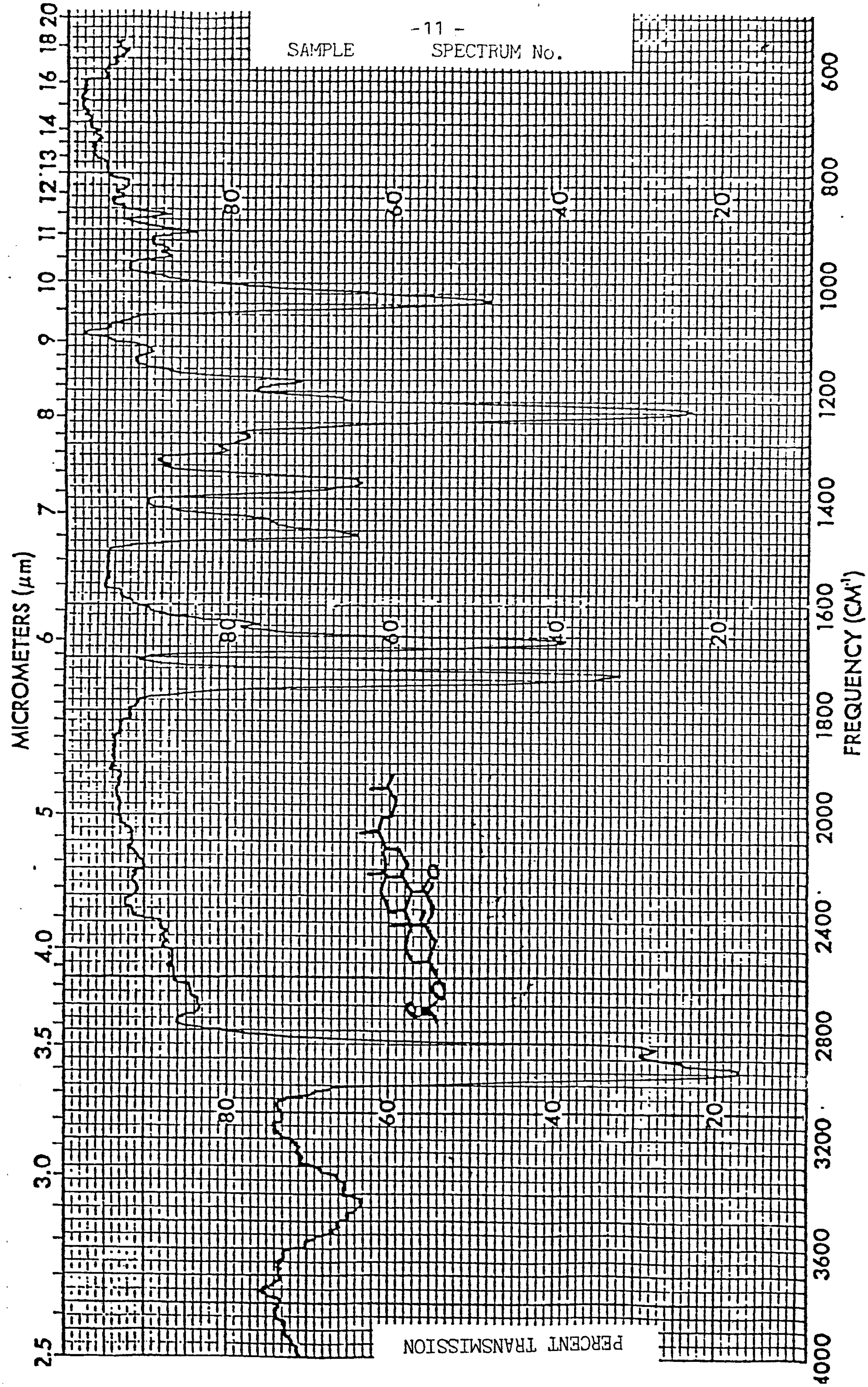
SAMPLE
SPECTRUM No.
MICROMETERS (μm)
FREQUENCY (CM⁻¹)
PERCENT TRANSMISSION

Beispiel III: Darstellung von 7ß-Hydroxy-cholesterol
($\Delta^5$-Cholesten-3ß, 7ß-diol)

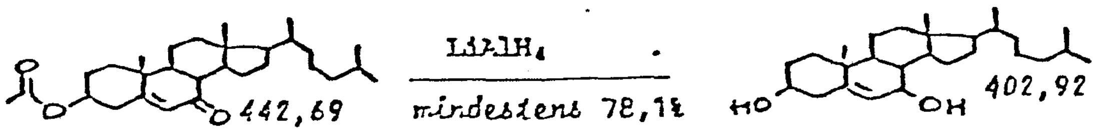


Smp. 159°C    Smp. 178°C

Anmerkungen:

Die Variante der Reduktion mit Aluminium-isopropylat nach Meerwein-Ponndorf von Windaus, Lettre und Schenk: Liebigs Ann. 520. 98 (1935) bzw. von O. Wintersteiner, W. L. Ruigh: J. Amer. chem. Soc. 64, 2453 (1942) liefert lediglich 20 % des 7ß-Epimeren aber ca. 80 % des physiologisch inaktiven 7α -Hydroxy-cholesterols.

Es wurde gefunden, daß die Reduktion des 7-Keto-cholesteryl acetats mit $LiAlH_4$, wesentlich stereospezifischer verläuft als die Reduktion nach Meerwein-Ponndorf. Das Hauptprodukt ist mit etwa 75 % Ausbeute das 7ß-Hydroxy-cholesterol; nur etwa 25 % des 7α -Epimeren werden gebildet, wie sich aus der optischen Drehung in $CHCl_3$ abschätzen läßt und was durch das $^{13}C$-Spektrum bestätigt wird.

Lt. Wintersteiner und Ritzmann, J. Biol.Chem. 136, 697 (1940) soll reines 7ß-Hydroxy-cholesterol den Smp. 154 - 157°C besitzen. Diese Angabe ist mit hoher Wahrscheinlichkeit falsch, denn aus Äther kristallisiertes 7ß-Hydroxy-cholesterol schmilzt bei 178,5°C. Aus viel $CH_3OH$ kristallisiert es mit einem Mol $CH_3OH$ und zeigt den Smp. 186°C. Diese Modifikation scheint sehr selten aufzutreten, denn gewöhnlich besitzt aus $CH_3OH$ umkristallisiertes 7ß-Hydroxy-cholesterol den Smp. 177-178,5°C; es soll Mischkristalle verschiedener Schmelzpunkte und $CH_3OH$-Gehalte mit der hochschmelzenden Modifikation bilden.

Das rohe, aus $CH_3OH$ kristallisierende Gemisch der beiden epimeren Diole (Smp. 173 - 174°C; $(\alpha)_D$ -14,3°/1,33 % in $CHCl_3$) verändert seinen Schmelzpunkt nicht, wenn es aus Aceton umkristallisiert wird. Dgl. gilt für das Umkristallisieren aus kleinen Mengen $CH_3OH$.

7α-Hydroxy-cholesterol ist eine stark links drehende Substanz ( $(\alpha)_D^{23}$ -87,6° / 0,96% in $CHCl_3$). Die höchste spezifische Rotation, die für die $CH_3OH$-haltige Modifikation des 7α-Hydroxy-cholesterols gemessen wurde, betrug -91,2°.
Sie ist wesentlich leichter in organischen Lösungsmitteln löslich als das 7ß-Epimere und ist nur schwierig in kristalliner Form zu erhalten. Kristallisiertes 7α-Hydroxy-cholesterol ist fast immer mit 7ß-Hydroxy-cholesterol verunreinigt.

7ß-Hydroxy-cholesterol besitzt den spezifischen Drehwert von $(\alpha)_D$ + 4,6 in $CHCl_3$, stellt also eine geringfügig rechts drehende Substanz dar.

Die spezifische Drehung ist deshalb ein besseres Kriterium der Reinheit als der Schmelzpunkt, der u. U. fehlerhafte Veränderungen bei der Kristallisation ergeben kann, sofern die stabilere niedrig-schmelzende Modifikation einmal anwesend ist.

Die Reinheit des Präparates kann jedoch auch unter dem Schmelzpunktmikroskop kontrolliert werden: reines 7ß-Hydroxy-cholesterol, das aus Äther erhalten wurde, zeigt nämlich die Eigentümlichkeit, etwa 10° unterhalb des Smp. von 177 - 178,5°C lange, verfilzte Fäden auswachsen zu lassen, die auch nach dem Erkalten des geschmolzenen Präparates wieder erscheinen. 7α-Hydroxy-cholesterol zeigt diese Eigenschaft nicht.

22,1 g (= 0,05 Mol) reines 7-Keto-cholesteryl-acetat vom Smp. 157 - 159 °C wurde in 500 ml über metallischem Natrium getrocknetem, peroxidfreiem,Äther heiß gelöst (Rückflußkühler) und auf Zimmertemperatur abgekühlt.

In einem (1) Liter-Dreihalskolben mit Rührer, Rückflußkühler und Tropftrichter wurde eine Lösung von 2,5 g(= 0,05 Mol + 20 %) $LiAlH_4$ in 200 ml absolutem Äther vorgelegt.

Unter kräftigem Rühren wurde sodann aus dem Tropftrichter die obige ätherische Lösung von 7-Keto-cholesteryl-acetat in dünnem Strahl so zugesetzt,daß der Äther gerade am Sieden erhalten wurde. Nach beendeter Zugabe, die etwa 15 bis 20 Minuten in Anspruch nahm, wurde noch eine (1) Stunde unter Rühren erhitzt und die Lösung unter Rühren erkalten gelassen.

Sodann wurde in Eiswasser gekühlt und so lange, Tropfen für Tropfen, mit $H_2O$ versetzt, wie sich noch $H_2$ entwickelt, der mittels eines Schlauches in den Abzugskamin geleitet wird; $H_2O$-Verbrauch im Überschuß etwa 25 ml. Bei größeren Ansätzen statt des $H_2O$'s besser Essigsäureäthylester verwenden.

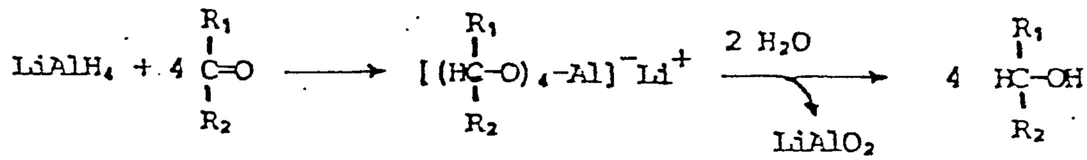

Zwecks Auflösung des entstandenen $LiAlO_2$'s wurde anschließend mit 80 ml 10%iger $H_2SO_4$ verrührt und nach der Überführung in einen 2000 ml Scheidetrichter mit 300 ml Äther verdünnt und durchgeschüttelt. Dabei geht das u.U. mikrokristallin ausgefallene Reaktionsprodukt vollständig in Lösung. Die abgetrennte saure, wäßrige Phase wurde einmal mit Äther nachextrahiert und die vereinigten ätherischen Lösungen mit 300 ml gesättigter NaCl-Lösung in zwei Portionen von je 150 ml gewaschen. Nach dem Trocknen über wasserfreiem $Na_2SO_4$ wurde das Filtrat drei (3) Stunden lang im Kühlschrank bei -1°C aufbewahrt. Der fadenförmig-verfilzte, fast schleimig, aussehende Niederschlag, der zuletzt den gesamten Kolben ausfüllte, wurde abgesaugt und wog nach dem Trocknen 5,8 g (= 28,85 % d.Th.); Smp. 177-178,5°C.
Die Mutterlauge wurde auf ca. 200ml eingeengt; nach mehrstündigem Stehen im Kühlschrank wurden 9,9 g (= 49,25 % d.Th) weißer Kristalle vom Smp. 177-178,5°C erhalten. Damit ergibt sich eine Gesamtausbeute von 15,7 g (= 78,1 % d.Th.).

Die restliche Mutterlauge wurde im Vakuum auf dem Wasserbade zur Trockne eingedampft. Der im Kolben verbliebene Rückstand wog 3,3 g (= 16,4 % d.Th.) und war von gelblichem Aussehen. Die Substanz schmilzt bei 157-159°C.

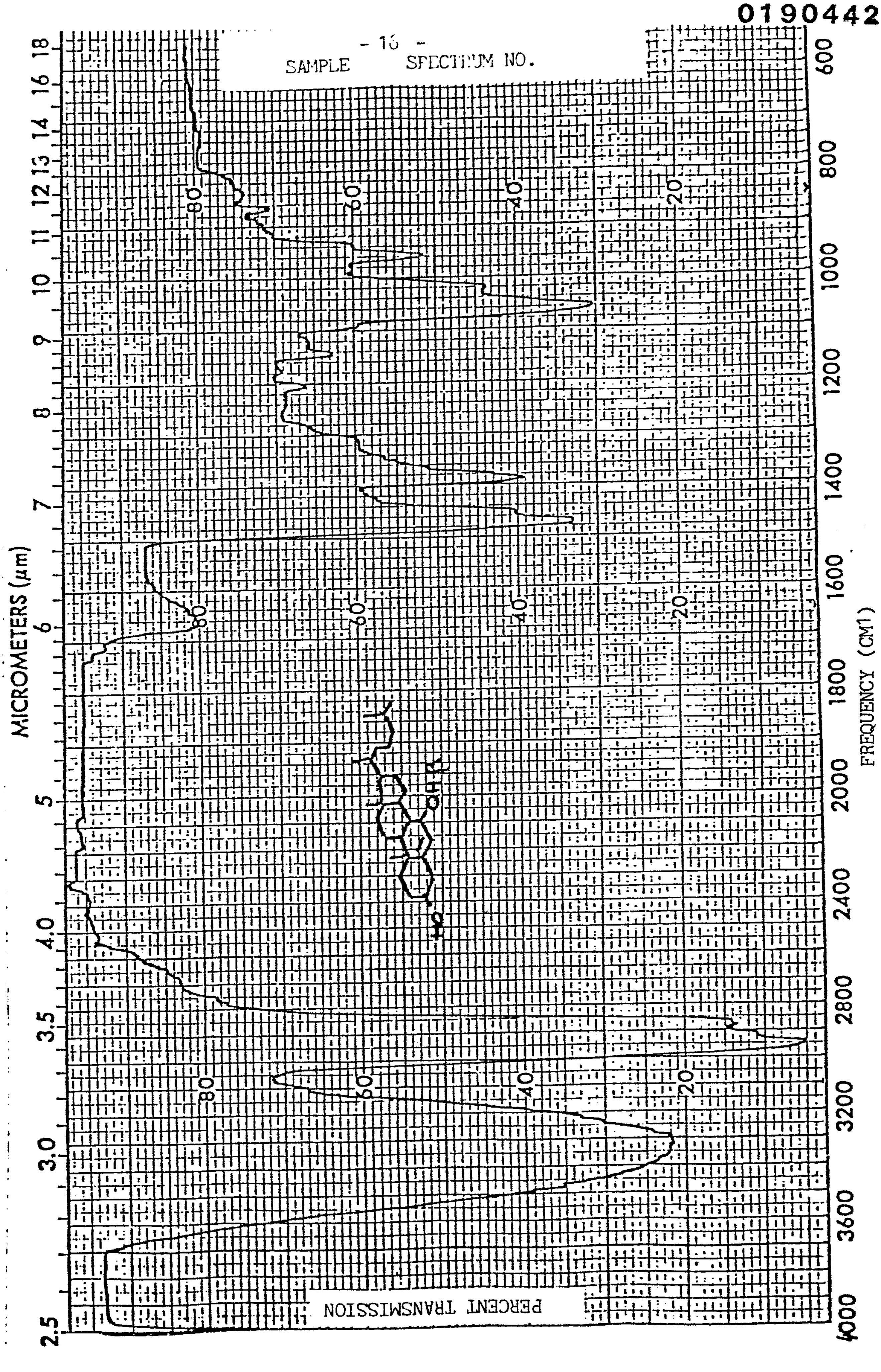
SAMPLE
SPECTRUM NO.
MICROMETERS (μm)
FREQUENCY (CM-1)
PERCENT TRANSMISSION

Ansprüche

1. Verfahren zur Darstellung von $\Delta^5$-Cholesten-3ß,7α,ß-diolen (7α,ß-Hydroxy-cholesterolen) und/oder $\Delta^{5,25}$-Cholestadien-3ß, 7α, ß-diolen (7α,ß-Hydroxy-desmosterolen), dadurch gekennzeichnet, daß die als Zwischenstufen verwendeten 3ß-Acetoxy-7-keto-$\Delta^5$-cholestene bzw. -$\Delta^{5,25}$-cholestene unter Verwendung von tert.-Butylchromat aus Cholesterol bzw. Desmosterol synthetisiert werden.

2. Verfahren nach Anspruch 1, dadurch gekennzeichnet, daß die Ausbeuten an den 3ß-Acetoxy-Zwischenstufen der unter 1. genannten 7-Ketone mindestens 62,3 % der Theorie betragen.

3. Verfahren nach Anspruch 1 und 2, dadurch gekennzeichnet, daß die Reduktion der 7-Keto-Gruppe statt mit Aluminium-isopropylat mittels Lithium-Aluminium-Hydrid vorgenommen wird.

4. Verfahren nach Anspruch 1 bis 3, dadurch gekennzeichnet, daß die Verwendung von Metallhydriden des Bors und des Aluminiums bei der Reduktion von 7-Keto-steroiden des hier beanspruchten Typs stereospezifischer zu Gunsten der 7ß-Hydroxylierung eingesetzt werden können als beispielsweise das bei der Meerwein-Ponndorf-Reduktionsmethode verwendete Aluminium-isopropylat.

5. Verfahren nach Anspruch 1 bis 4, dadurch gekennzeichnet, daß die stereospezifische Reduktion mittels der Hydride des Bors und des Aluminiums, beispielsweise Kaliumborhydrid oder Lithiumaluminiumhydrid, in Äthern, beispielsweise Diäthyläther, vorgenommen werden kann, worin die 7α-Epimeren wesentlich leichter löslich sind, als die 7 ß-Epimeren, wodurch eine einfache Trennung der beiden Epimeren möglich ist.

6. Verfahren nach Anspruch 1 bis 5, dadurch gekennzeichnet, daß die Trennung der beiden epimeren 7α - und 7 ß - hydroxylierten Abkömmlinge des Cholesterols bzw. des Desmosterols auch säulenchromatographisch erfolgen kann.

7. Verwendung der nach Anspruch 1 bis 6 dargestellten 7ß-hydroxylierten Stenole als native, im Warmblütler-Tierreich universelle, bio-chemische und daher unschädliche Signalsubstanzen zur Modulation der Immunantwort bei Krebserkrankungen aller Phänotypen.

0190442

Nummer der Anmeldung

Europäisches Patentamt

# EUROPÄISCHER RECHERCHENBERICHT

EP 85 11 5828

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| | | | C 07 J 9/00 |
| X | CHEMICAL ABSTRACTS, Band 44, Nr. 9, 10. Mai 1950, Seite 3971, Nr. 3871c, Columbus, Ohio, US; R.V. OPPENHAUER et al.: "A new oxidant", & ANALES ASOC. QUIM. ARGENTINA 1949, 37, 246-62 * Zusammenfassung * | 1 | |
| X | CHEMICAL ABSTRACTS, Band 78, Nr. 11, 19. März 1973, Seite 502, Nr. 72423y, Columbus, Ohio, US; T. KOLEK et al.: "Products of oxidation of delta5-steroids with tertiary butyl chromate", & BULL. ACAD. POL SCR., SER. SCI. CHIM. 1972, 20(11-12) 1009-13 * Zusammenfassung * | 1 | |
| | | | **RECHERCHIERTE SACHGEBIETE (Int. Cl 4)** |
| A | GB-A- 454 260 (ARTHUR CARPMAEL) * Seite 3, Beispiel 2; Seite 4, Ansprüche * | 1,3,4 | C 07 J 9/00 |
| A | US-A-4 157 391 (FUMIO KITUME) * Ansprüche 1,2 * | 7 | |

Der vorliegende Recherchenbericht wurde für alle Patentansprüche erstellt.

| Recherchenort | Abschlußdatum der Recherche | Prüfer |
|---|---|---|
| DEN HAAG | 09-04-1986 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82